# EUROPÄISCHE PATENTANMELDUNG

(11) **EP 3 572 765 A1**
(43) Veröffentlichungstag der Anmeldung: **27.11.2019**
(21) Anmeldenummer: 18173781.8
(22) Anmeldetag: 23.05.2018
(51) Int. Cl.: G01B 9/02, A61B 3/10

(54) **OCT-SYSTEM UND OCT-VERFAHREN**

(71) Anmelder: HAAG-STREIT AG, CH-3098 Köniz (CH)
(72) Erfinder: STALDER, Peter, 3504 Niederhüningen (CH); ROBLEDO, Lucio, 3013 Bern (CH)
(74) Vertreter: Glawe, Delfs, Moll

(57) **Zusammenfassung**

OCT-System mit einer OCT-Lichtquelle (16) zum Aussenden von OCT-Licht (15) in einen Objektstrahlengang (23) und einen Referenzstrahlengang (24). Das System umfasst einen Detektor (25, 52, 53) zum Aufnehmen eines aus dem Objektstrahlengang (23) und dem Referenzstrahlengang (24) erzeugten Interferenzsignals. In dem Objektstrahlengang (23) ist ein polarisationsabhängiges Verzögerungselement (30) angeordnet. Die Erfindung betrifft außerdem ein zugehöriges OCT-Verfahren. Mit der Erfindung können die Auswirkungen parasitärer Reflexionen vermindert werden.

## Beschreibung

Die Erfindung betrifft ein OCT-System mit einer OCT-Lichtquelle zum Aussenden von OCT-Licht in einen Objektstrahlengang und einen Referenzstrahlengang. Mit einem Detektor wird ein aus dem Objektstrahlengang und dem Referenzstrahlengang erzeugtes Interferenzsignal aufgenommen. Die Erfindung betrifft außerdem ein OCT-Verfahren.

Bei der optischen Kohärenztomographie (OCT) handelt es sich um ein bildgebendes Messverfahren. OCT-Licht wird auf ein Objekt, insbesondere menschliches Gewebe geleitet. Aus den zurückgeworfenen Anteilen des Lichts wird auf Streuzentren in dem Objekt geschlossen. Dazu wird der von dem Objekt zurückgeworfene Objektstrahlengang mit einem Referenz-Strahlengang überlagert. Die Bildinformation wird durch Auswerten des Interferenzsignals der beiden Strahlengänge gewonnen.

Es kann vorkommen, dass das Interferenzsignal durch parasitäre Reflexionen gestört ist. Dies bedeutet, dass das Interferenzsignal nicht nur aus von dem zu untersuchenden Objekt zurückgeworfenen Licht resultiert, sondern dass das Interferenzsignal auch durch andere Lichtreflexe beeinflusst ist, beispielsweise Lichtreflexe von optischen Komponenten im OCT-Strahlengang. Durch solche parasitären Reflexionen kann die Qualität des OCT-Signals beeinträchtigt werden.

Aus US 2015/173607 A1 ist bekannt, in einem ersten Schritt eine Messung mit Objekt im Messbereich aufzunehmen und in einem zweiten Schritt eine Messung ohne Objekt im Messbereich aufzunehmen. Durch Differenzbildung zwischen den beiden Messungen können die durch parasitäre Reflexionen verursachten Artefakte entfernt werden.

Der Erfindung liegt die Aufgabe zugrunde, ein OCT-System und ein OCT-Verfahren vorzustellen, bei denen der störende Einfluss parasitärer Reflexionen vermindert ist. Ausgehend vom genannten Stand der Technik wird die Aufgabe gelöst mit den Merkmalen der unabhängigen Ansprüche. Vorteilhafte Ausführungsformen sind in den Unteransprüchen angegeben.

Bei dem erfindungsgemäßen OCT-System ist in dem Objektstrahlengang ein polarisationsabhängiges Verzögerungselement angeordnet.

Bei einem polarisationsabhängigen Verzögerungselement wird durchtretendes Licht in Abhängigkeit vom Polarisationszustand unterschiedlich verzögert. Die Erfindung hat erkannt, dass es durch Anordnen eines polarisationsabhängigen Verzögerungselements im Objektstrahlengang möglich wird, das vom Objekt zurückgeworfene Licht von parasitären Reflexionen zu unterscheiden. Die parasitären Reflexionen, die nicht durch das polarisationsabhängige Verzögerungselement hindurchgetreten sind, unterscheiden sich in ihrem Polarisationszustand von dem Lichtsignal, das das polarisationsabhängige Verzögerungselement durchquert hat. Der Polarisationszustand im Referenzstrahlengang kann so eingestellt werden, dass das vom Objekt zurückgeworfene Licht stark zum Interferenzsignal beiträgt, während der Einfluss der parasitären Reflexionen gering ist.

Das Licht aus dem Referenzstrahlengang kann an einem Interferenz-Strahlteiler mit Licht aus dem Objektstrahlengang zur Interferenz gebracht werden. Das polarisationsabhängige Verzögerungselement kann so eingerichtet sein, dass am Interferenz-Strahlteiler der Polarisations-Überlapp zwischen dem vom Messobjekt zurückgestreuten Licht und dem aus dem Referenzstrahlengang eintreffenden Licht größer ist als der Polarisations-Überlapp zwischen dem von einem oder mehreren optischen Elementen im Objektstrahlengang reflektierten Licht und dem Referenzstrahlengang eintreffenden Licht. Der Begriff Polarisations-Überlapp bezieht sich auf die Ähnlichkeit zweier Polarisationszustände. Ein reiner Polarisationszustand lässt sich durch seinen Jones-Vektor repräsentieren: e=[a1e^{iθ1}; a2e^{iθ2}], wobei a1 und a2 die Amplituden und θ1 und θ2 die Phasen der beiden Komponenten des elektrischen Feldvektors beschreiben. Der Überlapp zwischen zwei Polarisationszuständen ist dann der Absolutbetrag des Quadrats des inneren Produkts zwischen den normierten Jones-Vektoren dieser beiden Polarisationszustände. Somit ist der Überlapp zwischen orthogonalen Polarisationszuständen (beispielsweise zwischen linear horizontal und linear vertikal polarisiertem Licht, oder zwischen links- und rechts-zirkular polarisiertem Licht) gleich null. Der Überlapp zwischen linear polarisiertem Licht und zirkular polarisiertem Licht ist 0.5, und der Überlapp zwischen zwei gleichen Polarisationszuständen ist 1. Der Polarisations-Überlapp zwischen dem vom Messobjekt zurückgestreuten Licht und dem aus dem Referenzstrahlengang eintreffenden Licht kann insbesondere um wenigstens 0.2, vorzugsweise um wenigstens 0.5, weiter vorzugsweise um wenigstens 0.8 größer sein als der Polarisations-Überlapp zwischen von einem oder mehreren optischen Elementen im Objektstrahlengang reflektiertem Licht und dem aus dem Referenzstrahlengang eintreffenden Licht.
Es ist von Vorteil, wenn das OCT-Licht vor dem Eintreten in das Verzögerungselement polarisiert ist. Insbesondere kann das OCT-Licht einen reinen Polarisationszustand haben mit einem Polarisationsgrad von beispielsweise wenigstens 80 %, vorzugsweise von wenigstens 90 % ist. In einer Ausführungsform ist das OCT-Licht vor dem Eintreten in das Verzögerungselement linear polarisiert.

Das OCT-System kann eine polarisierte Lichtquelle umfassen, so dass das von der Lichtquelle ausgehende OCT-Licht bereits einen polarisierten Zustand hat. Zusätzlich oder alternativ dazu kann zwischen der OCT-Lichtquelle und dem Verzögerungselement ein Polarisationsfilter angeordnet sein. Als Polarisationsfilter wird allgemein eine optische Einrichtung bezeichnet, durch die Licht nur in polarisiertem Zustand hindurchtreten kann.

Zusätzlich oder alternativ dazu kann zwischen dem der OCT-Lichtquelle und dem polarisationsabhängigen Verzögerungselement ein erster Polarisationssteller angeordnet sein. Als Polarisationssteller wird allgemein eine Einrichtung bezeichnet, mit der der Polarisationszustand von Licht gezielt eingestellt werden kann. Ein Polarisationssteller kann als statischer oder als variabler Polarisationssteller ausgebildet sein. Bei einem statischen Polarisationssteller wird der Polarisationszustand einmal justiert und dann nicht mehr verändert. Bei einem variablen Polarisationssteller kann der Polarisationszustand im Betrieb eingestellt werden. Ein variabler Polarisationssteller kann beispielsweise dadurch verwirklicht werden, dass ein Lichtleiter des betreffenden OCT-Lichtwegs in einer oder mehreren Windungen gelegt wird und diese Windungen relativ zu anderen Abschnitten des Lichtleiters mechanisch schwenkbar sind. Insbesondere kann der Lichtleiter mehrere Sätze von Windungen umfassen, die unabhängig voneinander geschwenkt werden können. Bei geeigneter Gestaltung des variablen Polarisationsstellers lassen sich auf diese Weise beliebige Wechsel zwischen reinen Polarisationszuständen einstellen. Der Polarisationssteller kann für eine Handbetätigung ausgelegt sein, so dass der Polarisationszustand manuell von einer Bedienperson eingestellt werden kann. Möglich ist auch ein motorischer Antrieb des Polarisationsstellers. Der erste Positionssteller kann genutzt werden, um das in das polarisationsabhängige Verzögerungselement eintretende OCT-Licht in einen gewünschten Polarisationszustand zu bringen.

Der Polarisationsfilter und/oder der erste Polarisationssteller können zwischen der OCT-Lichtquelle und einem Strahlenteiler angeordnet sein, in dem das OCT-Licht in einen Objektstrahlengang und einen Referenzstrahlengang aufgespalten wird. Der Strahlenteiler kann so gestaltet sein, dass 50% der eingangsseitigen Leistung in den Referenzstrahlengang und 50% in den Objektstrahlengang geleitet werden. Der Strahlenteiler kann beispielsweise als Faserkoppler ausgebildet sein, an den ein von der OCT-Lichtquelle kommender erster Lichtleiter, ein zu dem Objektstrahlengang gehöriger zweiter Lichtleiter und ein zu dem Referenzstrahlengang gehöriger dritter Lichtleiter angeschlossen sind.

Das polarisationsabhängige Verzögerungselement kann so angeordnet sein, dass es von dem OCT-Licht im Objektstrahlengang zweimal durchquert wird. Die Ausbreitungsrichtung des OCT-Lichts beim ersten Durchqueren kann entgegengesetzt zu der Ausbreitungsrichtung beim zweiten Durchqueren sein. Insbesondere kann das OCT-Licht einmal auf dem Hinweg zum Messobjekt und einmal auf dem Rückweg vom Messobjekt kommend durch das Verzögerungselement hindurchtreten.

Das Verzögerungselement kann so gestaltet sein, dass der Polarisationszustand nach dem Durchqueren des Verzögerungselements orthogonal zu dem Polarisationszustand vor dem Eintreten in das Verzögerungselement ist. Es kann dann ein Interferenzsignal erzeugt werden, in dem der Beitrag des vom Messobjekt kommenden Lichtsignals maximal und der Beitrag der parasitären Reflexionen minimal ist. Tritt der Objektstrahlengang zweimal durch das Verzögerungselement hindurch, so kann der orthogonale Polarisationszustand sich als Summe aus den beiden Durchtritten ergeben. Beispielsweise kann das Verzögerungselement als λ/4-Plättchen ausgestaltet sein. Das Verzögerungselement kann so ausgerichtet sein, dass die beim ersten Eintritt lineare Polarisation in eine zirkulare Polarisation gedreht wird, so dass das vom Messobjekt zurückgestreute Licht beim zweiten Durchlaufen des Verzögerungselements in eine orthogonale lineare Polarisation gedreht wird. Nach dem Durchqueren des Verzögerungselements kann der Objektstrahlengang ohne weitere Veränderung des Polarisationszustands zur Interferenz mit dem Referenzstrahlengang gebracht werden.

Das OCT-System kann so gestaltet sein, dass alle optischen Elemente, die den Objektstrahlengang formen oder umlenken, zwischen der OCT-Lichtquelle und dem Verzögerungselement angeordnet sind. Wenn der Objektstrahlengang sich zwischen dem Verzögerungselement und dem Messobjekt frei ausbreiten kann, können in diesem Abschnitt des Objektstrahlengangs keine weiteren parasitären Reflexionen entstehen. Da die vor dem Verzögerungselement entstehenden parasitären Reflexionen aus dem Interferenzsignal ausgeblendet werden können, lässt sich auf diese Weise eine gute Qualität des Messsignals erreichen.

Auch in dem Verzögerungselement selbst können parasitäre Reflexionen entstehen. Um zu verhindern, dass diese das Interferenzsignal stören, kann das Verzögerungselement so angeordnet sein, dass eine oder mehrere Grenzflächen, die vom Objektstrahlengang durchquert werden, relativ zu dem Objektstrahlengang gekippt sind. Dies kann insbesondere die Grenzfläche betreffen, über die der Objektstrahlengang in Richtung des Messobjekts aus dem Verzögerungselement austritt bzw. vom Messobjekt kommend in das Verzögerungselement eintritt. Wenn eine Grenzfläche relativ zum Objektstrahlengang gekippt ist, trifft der Objektstrahlengang nicht orthogonal, sondern unter einem anderen Winkel auf die Grenzfläche. Parasitäre Reflexionen werden zur Seite abgelenkt und stören das Interferenzsignal nicht.

Im Referenzstrahlengang kann ein zweiter Polarisationssteller angeordnet sein, so dass der Polarisationszustand des OCT-Lichts im Referenzstrahlengang an den Polarisationszustand des vom Messobjekt kommenden OCT-Lichts angepasst werden kann. Anpassen bedeutet, dass der Polarisationszustand im Referenzstrahlengang so eingestellt wird, dass die Interferenz mit dem vom Messobjekt kommenden OCT-Licht maximal wird. Der Polarisationssteller kann ein statischer Polarisationssteller oder ein dynamischer Polarisationssteller sein. Ist das OCT-Licht beispielsweise linear polarisiert, so können das vom Messobjekt kommende OCT-Licht und das damit zur Interferenz gebrachte OCT-Licht des Referenzstrahlengangs in zueinander parallele Polarisationszustände gebracht werden.

Das OCT-System kann einen oder mehrere Lichtleiter umfassen, durch die das OCT-Licht hindurchtritt. Die Lichtleiter können als Monomoden-Lichtleiter ausgebildet sein, um die Entstehung von Störsignalen innerhalb der Lichtleiter zu vermeiden. Das OCT-System kann einen ersten Faserkoppler umfassen, in dem das OCT-Licht in einen Objektstrahlengang und einen Referenzstrahlengang aufgespalten wird. In dem ersten Faserkoppler kann auch das Interferenzsignal erzeugt werden. Die Ausbreitungsrichtung beim Aufspalten des OCT-Lichts kann entgegengesetzt sein zu der Ausbreitungsrichtung beim Erzeugen des Interferenzsignals.

Möglich ist auch, dass das OCT-System einen zweiten Faserkoppler umfasst, in dem das Interferenzsignal erzeugt wird. Der Referenzstrahlengang kann sich zwischen dem ersten Faserkoppler und dem zweiten Faserkoppler erstrecken. Zwischen dem ersten Faserkoppler und dem zweiten Faserkoppler kann ein Lichtleiter angeordnet sein, in dem der Referenzstrahlengang geführt ist. Die Länge des Referenzstrahlengangs kann der Länge des optischen Wegs zwischen dem ersten Faserkoppler und dem zweiten Faserkoppler entsprechen.

Das OCT-System kann einen vierten Lichtleiter umfassen, der sich zwischen dem ersten Faserkoppler und dem zweiten Faserkoppler erstreckt, wobei der vierte Lichtleiter einen Abschnitt des Objektstrahlengangs bildet. Die Länge des Objektstrahlengangs insgesamt kann sich als Summe der Länge des vierten Lichtleiters sowie des doppelten Wegs zwischen dem ersten Faserkoppler und dem Objekt ergeben.

Konventionelle Lichtleiter haben die Eigenschaft, dass der Polarisationszustand des Lichts sich ändern kann, wenn die räumliche Konfiguration des Lichtleiters verändert wird. Beispielsweise kann es einen Einfluss auf den Polarisationszustand des Lichts haben, ob der Lichtleiter sich geradlinig oder entlang von Windungen erstreckt. Das OCT-System kann einen oder mehrere Polarisationssteller umfassen, um solche Änderungen des Polarisationszustands wieder auszugleichen.

Das OCT-System kann so gestaltet sein, dass eine oder mehrere Lichtleiter als polarisationserhaltende Lichtleiter ausgebildet sind. Als polarisationserhaltend wird ein Lichtleiter bezeichnet, wenn bezogen auf das Koordinatensystem des Lichtleiters die Polarisationszustände am Eingang und am Ausgang des Lichtleiters in fester Weise miteinander gekoppelt sind. Relativ zum Raum-Koordinatensystem kann der Polarisationszustand durch Ändern der räumlichen Konfiguration des polarisationserhaltenden Lichtleiters verändert werden. Ein polarisationserhaltender Lichtleiter kann eine erste Achse und eine zweite Achse aufweisen, in denen das Licht sich mit unterschiedlichen Ausbreitungsgeschwindigkeiten bewegt (langsame Achse/schnelle Achse). Die Verwendung von polarisationserhaltenden Lichtleitern kann sich insbesondere anbieten, wenn das OCT-System so gestaltet ist, dass im Betrieb die räumliche Anordnung von Komponenten des Systems relativ zueinander verändert werden kann. Dies ist regelmäßig mit einer Verformung von Lichtleitern verbunden, was bei konventionellen Lichtleitern einen Einfluss auf den Polarisationszustand des OCT-Lichts haben könnte.

Zwischen der OCT-Lichtquelle und dem ersten Faserkoppler, in dem die Aufspaltung in den Objektstrahlengang und den Referenzstrahlengang erfolgt, kann ein polarisationserhaltender Lichtleiter angeordnet sein. Der lineare Polarisationszustand des in den Lichtleiter eingespeisten OCT-Lichts kann parallel zu der ersten Achse des polarisationserhaltenden Lichtleiters ausgerichtet sein.

Ein erster Abschnitt des Objektstrahlengangs kann als polarisationserhaltender Lichtleiter ausgebildet sein. Der polarisationserhaltende Lichtleiter kann von dem OCT-Licht des Objektstrahlengangs zweimal mit entgegengesetzten Ausbreitungsrichtungen durchquert werden. Dabei kann das OCT-Licht sich auf dem Hinweg zum Objekt in der ersten Achse des polarisationserhaltenden Lichtleiters ausbreiten und auf dem Rückweg vom Objekt kommend in der zweiten Achse des polarisationserhaltenden Lichtleiters ausbreiten.

Ein zweiter Abschnitt des Objektstrahlengangs kann als polarisationserhaltender Lichtleiter ausgebildet sein. Der zweite Abschnitt des Objektstrahlengangs kann sich zwischen dem ersten Faserkoppler und dem zweiten Faserkoppler erstrecken. Im zweiten Abschnitt des Objektstrahlengangs kann das OCT-Licht sich in der zweiten Achse des polarisationserhaltenden Lichtleiters ausbreiten.

Der Referenzstrahlengang kann als polarisationserhaltender Lichtleiter ausgebildet sein. In einem ersten Abschnitt des Referenzstrahlengangs kann das OCT-Licht sich in der ersten Achse des polarisationserhaltenden Lichtleiters erstrecken. In einem zweiten Abschnitt des Referenzstrahlengangs kann das OCT-Licht sich in der zweiten Achse des polarisationserhaltenden Lichtleiters erstrecken. Ein Interferometer mit einem solchen Referenzstrahlengang hat eigenständigen erfinderischen Gehalt unabhängig davon, ob es in einem OCT-System verwendet wird und dass im Objektstrahlengang ein polarisationsabhängiges Verzögerungselement angeordnet ist.

Zwischen dem ersten Abschnitt und dem zweiten Abschnitt kann ein Faserverbinder angeordnet sein. In dem Faserverbinder kann das OCT-Licht von der ersten Achse in die zweite Achse übergeleitet werden. Dies kann dadurch geschehen, dass der erste Abschnitt und der zweite Abschnitt in dem Faserverbinder so gekoppelt sind, dass sie um 90° gegeneinander verdreht sind. Der Faserverbinder kann in Form einer Steckverbindung realisiert werden. Alternativ kann auch ein Faserspleiß zum Einsatz kommen, wobei die beiden zu verbindenden Faserenden mit einem Lichtbogen verschmolzen werden.

Die Länge des ersten Abschnitts und die Länge des zweiten Abschnitts des Referenzstrahlengangs können in einem Verhältnis zueinander stehen, das den Gegebenheiten im Objektstrahlengang entspricht, so dass das OCT-Licht in beiden Fällen denselben Weg in der ersten Achse und in der zweiten Achse zurücklegt. Im Sinne der Erfindung ist ein solcher Faserverbinder ein Polarisationssteller, der anfänglich justiert wird und dann nicht mehr verändert wird.

Das OCT-System kann mit in der Wellenlänge durchstimmbarer Lichtquelle ausgeführt werden (Swept-source OCT). Das OCT-System kann dann einen ersten Detektor und einen zweiten Detektor aufweisen, um die um 180° phasenverschobenen Interferenzsignale zu erfassen, die am zweiten Faserkoppler gebildet werden. Die Differenz der Photoströme kann in eine Spannung gewandelt und digitalisiert. Das Interferenzsignal für einen Durchstimmvorgang der Lichtquelle kann spektral aufgelöst digitalisiert werden und anschließend in ein räumliches Signal transformiert werden. Alternativ kann auch eine breitbandige Lichtquelle zum Einsatz kommen, als Detektor verwendet man dann ein Spektrometer (Spectral domain OCT).

Der Objektstrahlengang kann einen Abschnitt umfassen, in dem das OCT-Licht sich frei ausbreitet, also nicht innerhalb eines Lichtleiters geführt ist. Der Abschnitt kann sich zwischen einem Austrittsende eines Lichtleiters und dem Messobjekt erstrecken. Es kann eine Kollimationsoptik vorgesehen sein, so dass der Objektstrahlengang sich in einem Abschnitt in kollimiertem Zustand erstreckt. Das OCT-System kann ein Objektiv umfassen, so dass der Objektstrahlengang im Bereich des Messobjekts fokussiert ist. Das erfindungsgemäße polarisationsabhängige Verzögerungselement kann zwischen dem Objektiv und dem Messobjekt angeordnet sein.

Mit einer Scaneinrichtung kann der Objektstrahlengang in seitliche Richtung abgelenkt werden. Durch Ablenken in seitlicher Richtung können Schnittbilder des Messobjekts erzeugt werden. Wenn die Scaneinrichtung dazu ausgelegt ist, den Objektstrahlengang in zwei seitliche Richtungen abzulenken (zum Beispiel X-Richtung, Y-Richtung), kann aus einer Mehrzahl von Schnittbildern ein dreidimensionales Volumenbild zusammengesetzt werden.

Die Scaneinrichtung kann beispielsweise zwei Scanspiegel umfassen, die um zueinander orthogonale Achsen schwenkbar sind. Eine solche Anordnung von Scanspiegeln ist ein gängiges Beispiel einer Scaneinrichtung, mit der ein Messobjekt abgetastet werden kann. Die Scaneinrichtung kann zwischen einer Kollimationsoptik und einem Objektiv des Objektstrahlengangs angeordnet sein. Die Optik des Objektstrahlengangs kann telezentrisch ausgelegt sein, so dass die Scaneinrichtung in einem Brennpunkt des Objektivs angeordnet ist und der Strahlengang zwischen dem Objektiv und dem Messobjekt beim Scannen parallel verschoben wird.

Der Referenzstrahlengang kann einen Abschnitt umfassen, in dem das OCT-Licht sich frei ausbreitet, also nicht innerhalb eines Lichtleiters geführt ist. Der Abschnitt kann sich zwischen einem Austrittsende eines Lichtleiters und einem Spiegel erstrecken. Zwischen dem Austrittsende des Lichtleiters und dem Spiegel kann eine Kollimationsoptik angeordnet sein, so dass das OCT-Licht in kollimiertem Zustand auf den Spiegel trifft. In anderen Ausführungsformen kann der gesamte Referenzstrahlengang sich innerhalb ein oder mehrerer Lichtleiter erstrecken.

Die Lichtquelle des OCT-Systems kann eine Swept-Source-Lichtquelle sein, bei der schmalbandiges OCT-Licht innerhalb einer Durchstimmzeit über einen spektralen Durchstimmbereich durch gestimmt wird. Der Detektor kann Photodioden umfassen, die zeitaufgelöst das Interferenzsignal erfassen und somit indirekt eine spektrale Auflösung des Interferenzsignals ermöglichen. Der Photostrom der Photodioden kann in eine Spannung gewandelt und digitalisiert werden. Das Interferenzsignal für einen Durchstimmvorgang der Swept-Source-Lichtquelle kann spektral aufgelöst digitalisiert und anschließend in ein räumliches Signal transformiert werden. In Kombination mit der lateralen Ablenkung des Objektstrahlengangs durch die Scaneinrichtunng können Schnittbilder des Messobjekts erstellt werden.

Das OCT-System kann für Messungen am menschlichen Auge verwendet werden. Die Linienbreite der Lichtquelle kann so gewählt werden, dass Strukturen bis zu einem Abstand von 40 mm zum Referenzpunkt noch gut erfasst werden können. Als Referenzpunkt wird eine Position im Objektstrahlengang bezeichnet, für die die optische Weglänge von Lichtquelle bis zum Referenzpunkt und von dort wieder zurück bis zum Interferenzstrahlteiler gleich ist mit der optischen Weglänge von Lichtquelle über Referenzstrahlengang bis zum Interferenzstrahlteiler. Die Oszillationsfrequenz des Interferenzsignals (in spektraler Darstellung) ist ein Maß für den Abstand der lichtstreuenden Struktur zum Referenzpunkt. Ein Referenzpunkt des OCT-Systems kann vor dem menschlichen Auge angeordnet sein.

Da im Allgemeinen nicht zwischen positiven und negativen Frequenzen unterschieden werden kann, ist ein OCT-System auch für Strukturen empfindlich, die auf der dem Messgerät zugewandten Seite des Referenzpunkts liegen. Optische Elemente im Objektstrahlengang können denselben Abstand vom Referenzpunkt haben wie bestimmte Streuzentren des Auges, weswegen die parasitären Reflexionen an diesen optischen Elementen allgemein Störpotenzial haben.

Es können auch weitere als die genannten Komponenten im Strahlengang des OCT-Systems enthalten sein. Beispielsweise kann für Swept-Source-OCT zwischen der OCT-Lichtquelle und der Aufspaltung in Objektstrahlengang und Referenzstrahlengang mit einem Strahlteiler ein Anteil des OCT-Lichts ausgekoppelt werden, um daraus ein Takt-Signal zur Digitalisierung des Interferenzsignals zu generieren.

Die Erfindung betrifft außerdem ein OCT-Verfahren, bei dem OCT-Licht ausgesendet wird. Das OCT-Licht wird in einen Objektstrahlengang und einen Referenzstrahlengang aufgespalten. Mit einem Detektor wird ein aus dem Objektstrahlengang und dem Referenzstrahlengang erzeugtes Interferenzsignal aufgenommen. Das OCT-Licht wird durch ein in dem Objektstrahlengang angeordnetes polarisationsabhängiges Verzögerungselement geleitet.

Das Verfahren kann mit weiteren Merkmalen fortgebildet werden, die im Zusammenhang des erfindungsgemäßen Systems beschrieben sind. Das System kann mit weiteren Merkmalen fortgebildet werden, die im Zusammenhang des erfindungsgemäßen Verfahrens beschrieben sind.

Die Erfindung wird nachfolgend unter Bezugnahme auf die beigefügten Zeichnungen anhand vorteilhafter Ausführungsformen beispielhaft beschrieben. Es zeigen:
- Fig. 1:: eine erste Ausführungsform eines erfindungsgemäßen OCT-Systems;
- Fig. 2:: eine zweite Ausführungsform eines erfindungsgemäßen OCT-Systems.

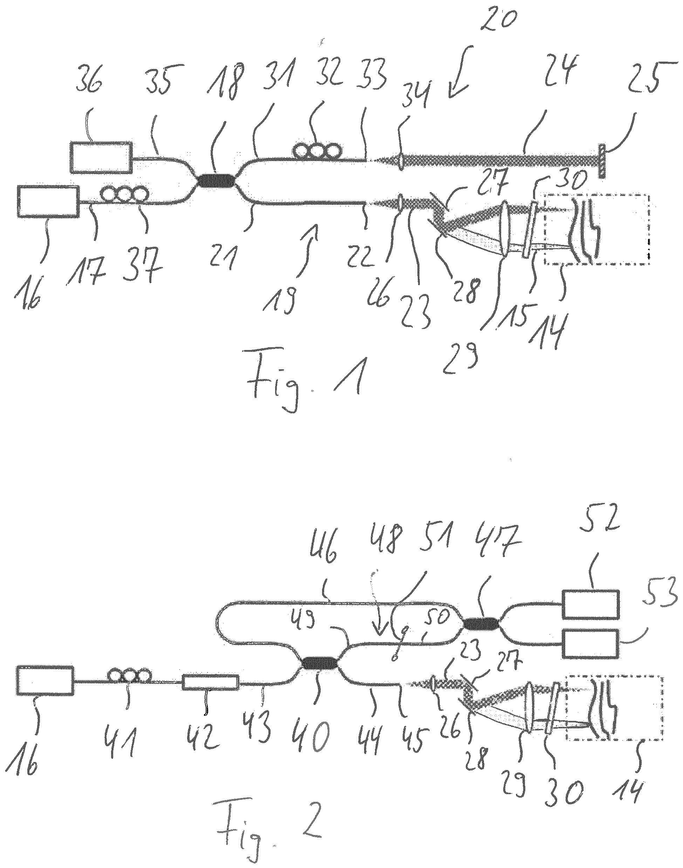

Ein in Fig. 1 gezeigtes OCT-System dient zur Untersuchung eines Messobjekts 14 in Form eines menschlichen Auges. Indem OCT-Licht 15 auf das Messobjekt 14 gerichtet wird, wird eine Bildinformation gewonnen, die sich entlang der Achse des OCT-Strahls in die Tiefe des Messobjekts 14 erstreckt. Indem der OCT-Strahl in einer dazu senkrechten Richtung über das Messobjekt 14 gescannt wird, kann aus einer Vielzahl von einzelnen Messaufnahmen ein dreidimensionales Bild des Messobjekts 14 gewonnen werden.

Das OCT-System umfasst eine OCT-Lichtquelle 16, die als Swept-Source-Lichtquelle ausgebildet ist. Die Swept-Source-Lichtquelle 16 erzeugt schmalbandiges Licht, das spektral durchstimmbar ist. Zu jedem Moment wird also schmalbandiges Licht ausgesendet, dessen Frequenz sich mit der Zeit ändert, so dass die Swept-Source-Lichtquelle während einer Durchstimmzeit über einen Frequenzbereich durchgestimmt wird.

Das von der OCT-Lichtquelle 16 abgegebene OCT-Licht 15 ist linear polarisiert und wird in einen ersten Lichtleiter 17 eingespeist, der als Monomoden-Lichtleiter ausgebildet ist. Der erste Lichtleiter 17 erstreckt sich zu einem Faserkoppler 18, in dem das OCT-Licht 15 aus dem ersten Lichtleiter 17 in einen Objektstrahlengang 23 und einen Referenz-Strahlengang 24 aufgespalten wird. Der Objektstrahlengang 23 erstreckt sich entlang einem Objektarm 19 bis zu dem Messobjekt 14. Der Referenzstrahlengang 24 erstreckt sich entlang einem Referenzarm 20 bis zu einem Referenzspiegel 25.

Der Objektarm 19 umfasst einen zweiten Lichtleiter 21, der sich von dem Faserkoppler 18 bis zur einem Austrittsende 22 erstreckt. An dem Austrittsende 22 tritt der Objektstrahlengang 23 in divergentem Zustand aus dem zweiten Lichtleiter 21 aus und wird mit einer Kollimationslinse 26 in einen kollimierten Zustand gebracht.

Eine Scaneinrichtung umfasst zwei Scanspiegel 27, 28, die um zwei zueinander orthogonale Achsen schwenkbar sind. Der Objektstrahlengang 23 wird über die Scaneinrichtung 27, 28 zu einem Objektiv 29 geleitet. Der Objektstrahlengang 23 tritt durch das Objektiv 29 hindurch und wird im Bereich des Messobjekts 14 fokussiert.

Durch Schwenken der Scanspiegel 27, 28 ändert sich die Richtung, unter der der Objektstrahlengang 23 auf das Objektiv 29 auftrifft. Da der zweite Scanspiegel 28 im Brennpunkt des Objektivs 29 angeordnet ist, erstreckt der Strahlengang 23 sich zwischen dem Objektiv 29 und dem Messobjekt 14 unabhängig von der Stellung des Scaneinrichtung 27, 28 parallel zur optischen Achse des Objektivs 29. Zwischen dem Objektiv 29 und dem Messobjekt 14 durchquert der Objektstrahlengang 23 ein polarisationsabhängiges Verzögerungselement 30 in Form eines A/4-Plättchens.

Von dem Messobjekt 14 zurückgeworfenes OCT-Licht bewegt sich mit entgegengesetzter Ausbreitungsrichtung entlang dem Objektarm 19 zurück bis zu dem Faserkoppler 18.

Der Referenzarm 20 umfasst einen dritten Lichtleiter 31, der sich von dem Faserkoppler 18 über einen zweiten Polarisationssteller 32 bis zu einem Austrittsende 33 erstreckt. Der in divergentem Zustand aus dem Austrittsende 33 austretende Referenzstrahlengang 24 trifft auf eine Kollimationslinse 34. Von der Kollimationslinse 34 breitet der Referenzstrahlengang 24 sich in kollimiertem Zustand bis zu dem Referenzspiegel 25 aus. Das von dem Referenzspiegel 25 reflektierte OCT-Licht bewegt sich mit entgegengesetzter Ausbreitungsrichtung entlang dem Referenzarm 20 zurück zu dem Faserkoppler 18.

Der Referenzspiegel 25 ist so angeordnet, dass der optische Weg zwischen dem Faserkoppler 18 und dem Referenzspiegel 25 im Referenzarm 20 genauso lang ist wie der optische Weg im Objektarm 19 zwischen dem Faserkoppler 18 und einem Referenzpunkt im Messobjekt 14. Da das OCT-Licht entlang dem Objektarm 19 und dem Referenzarm 20 denselben optischen Weg zurückgelegt hat, entsteht ein Interferenzsignal, wenn der Objektstrahlengang 23 und der Referenzstrahlengang 24 in dem Faserkoppler 18 wieder zusammengeführt werden. Das Interferenzsignal ist desto stärker, je mehr OCT-Licht von einer bestimmten Struktur innerhalb des Messobjekts 14 zurückgeworfen wird. Durch Auswerten des Interferenzsignals können also Streuzentren innerhalb des Messobjekts 14 identifiziert werden.

Ist ein Streuzentrum gerade an dem Referenzpunkt des Objektstrahlengangs angeordnet, so sind der optische Weg des Objektstrahlengangs 23 und des Referenzstrahlengangs 24 exakt gleich lang, so dass sich ein stehendes Interferenzsignal ergibt. Ist das Streuzentrum von dem Referenzpunkt entfernt, so oszilliert das Interferenzsignal (in spektraler Darstellung), wobei die Frequenz je größer wird, desto größer der Abstand zum Referenzpunkt ist.

Über einen vierten Lichtleiter 35 wird das Interferenzsignal zu einem Detektor 36 geleitet. Mit dem Detektor 36 wird das Interferenzsignal aufgenommen und in eine ortsaufgelöste Bildinformation umgerechnet.

Die Linienbreite der OCT-Lichtquelle 16, also die momentane spektrale Breite des abgegebenen Lichts, ist so klein, dass Strukturen noch gut erfasst werden können, die einen Abstand von beispielsweise 40 mm zum Referenzpunkt haben. Mit einem solchen Messbereich wird es möglich, mit dem erfindungsgemäßen OCT-System Aufnahmen eines menschlichen Auges zu machen. Dabei kann der Referenzpunkt knapp vor dem Auge liegen, so dass alle Strukturen des Auges jenseits des Referenzpunkt liegen. Da positive und negative Abstände vom Referenzpunkt im Interferenzsignal nicht unterschieden werden können, ist es wünschenswert, Reflexionen zu vermeiden, die vor dem Referenzpunkt angeordnet sind. Dies gilt insbesondere für Reflexionen an solchen optischen Elementen des Objektarms 19, deren Abstand vom Referenzpunkt kleiner ist als die Messtiefe des OCT-Systems. In dem Ausführungsbeispiel gemäß Fig. 1 können beispielsweise parasitäre Reflexionen von dem Verzögerungselement 30 oder von dem Objektiv 29 das Messsignal von dem Messobjekt 14 verfälschen.

Die Erfindung beruht auf dem Gedanken, die Auswirkungen solcher parasitären Reflexionen zu vermindern, indem das Messsignal in einen anderen Polarisationszustand versetzt wird als die parasitären Reflexionen. Durch geeignete Einstellung des Polarisationszustands im Referenzstrahlengang kann erreicht werden, dass sich aus dem OCT-Licht, das von dem Messobjekt 14 zurückgeworfen wird, ein maximales Interferenzsignal ergibt, während gleichzeitig das von den parasitären Reflexionen erzeugte Interferenzsignal minimal ist.

Mit einem ersten Polarisationssteller 37 wird der Polarisationszustand des von der OCT-Lichtquelle 16 abgegebenen OCT-Lichts so eingestellt, dass das an dem Austrittsende 22 des zweiten Lichtleiters 21 austretende Licht rein linear polarisiert ist. Der lineare Polarisationszustand bleibt bis zum Durchtritt durch das λ/4-Plättchen 30 erhalten. Das λ/4-Plättchen wird dabei so ausgerichtet (rotiert), dass nachdem das OCT-Licht zweimal mit entgegengesetzter Ausbreitungsrichtung durch das λ/4-Plättchen hindurchgetreten ist, das OCT-Licht weiterhin rein linear polarisiert, die Richtung der linearen Polarisation jedoch orthogonal zu der ursprünglichen linearen Polarisation ist. Dies ist typischerweise der Fall, wenn der Winkel zwischen linearer Polarisation am Eintritt in das Plättchen und der kristalloptischen Achse des Plättchens 45° beträgt.

Hingegen haben die parasitären Reflexionen von den optischen Elementen des Objektarms 19, die nicht durch das λ/4-Plättchen 30 hindurchgetreten sind, weiterhin den ursprünglichen linearen Polarisationszustand. Der lineare Polarisationszustand der parasitären Reflexionen ist also orthogonal bezogen auf den linearen Polarisationszustand des vom Messobjekt 14 zurückgeworfenen OCT-Lichts.

Mit dem zweiten Polarisationszustand 32 wird der lineare Polarisationszustand des Referenzstrahlengangs 24 so eingestellt, dass beim Überlagern des Objektstrahlengangs 23 und des Referenzstrahlengangs 24 im Faserkoppler 18 die lineare Polarisation des Referenzstrahlengangs 24 parallel zu der linearen Polarisation des von dem Messobjekt 14 kommenden OCT-Lichts ist. Damit erzeugt das von dem Messobjekt 14 zurückgeworfene OCT-Licht ein maximales Interferenzsignal, während gleichzeitig das von den parasitären Reflexionen erzeugte Interferenzsignal minimal ist.

Nicht beseitigt werden können auf diese Weise parasitäre Reflexionen, die auftreten, wenn das OCT-Licht in Richtung des Messobjekts 14 aus dem λ/4-Plättchen 30 austritt. Das λ/4-Plättchen 30 ist deswegen relativ zur optischen Achse des Objektivs 29 gekippt, so dass diese parasitären Reflexionen nicht in Richtung des Faserkopplers 18 geleitet werden, sondern zur Seite abgelenkt werden.

Bei dem Ausführungsbeispiel gemäß Fig. 2 ist die OCT-Lichtquelle 16 ebenfalls als Swept-Source-Lichtquelle ausgebildet, die linear polarisiertes OCT-Licht abgibt. Zwischen der OCT-Lichtquelle 16 und einem ersten Faserkoppler 40 sind ein Polarisationssteller 41 und ein Polarisationsfilter 42 angeordnet. Der Polarisationsfilter 42 ist so ausgelegt, dass er lediglich solches Licht hindurchtreten lässt, das in einer bestimmten Richtung linear polarisiert ist. Mit dem Polarisationssteller 41 wird der Polarisationszustand des OCT-Lichts so eingestellt, dass er mit der linearen Polarisationsrichtung des Polarisationsfilters 42 übereinstimmt. Mit anderen Worten wird der Polarisationssteller 41 so eingestellt, dass am Ausgang des Polarisationsfilters 42 die maximale Lichtmenge austritt.

Zwischen dem Polarisationsfilter 42 und dem ersten Faserkoppler 40 erstreckt sich ein erster Lichtleiter 43, der als polarisationserhaltender Lichtleiter ausgebildet ist. Der polarisationserhaltende Lichtleiter 43 ist so an den Polarisationsfilter 42 angeschlossen, dass das gesamte Licht in die schnelle Achse des Lichtleiters 43 eingespeist wird.

In dem ersten Faserkoppler 40 wird das OCT Licht in den Objektstrahlengang 23 und den Referenzstrahlengang 24 aufgespalten. Im Objektarm 19 erstreckt sich ein zweiter polarisationserhaltender Lichtleiter 44 zwischen dem ersten Faserkoppler 40 und einem Austrittsende 45. Der zweite polarisationserhaltende Lichtleiter 44 ist so an den ersten Faserkoppler 40 angeschlossen, dass die schnellen Achsen der Lichtleiter 43, 44 übereinstimmen. Das OCT-Licht aus der schnellen Achse des ersten polarisationserhaltenden Lichtleiters 43 geht also in die schnelle Achse des zweiten polarisationserhaltenden Lichtleiters 44 über.

Die Komponenten des Objektarms 19 zwischen dem Austrittsende 45 des Lichtleiters 44 und dem Messobjekt 14 sind identisch wie bei dem Ausführungsbeispiel gemäß Fig. 1. Die von dem Messobjekt 14 zurückgeworfenen Anteile des OCT-Lichts sind also zweimal durch das λ/4-Plättchen 30 hindurchgetreten. Das λ/4-Plättchen wird dabei so ausgerichtet (rotiert), dass der lineare Polarisationszustand des zurückgeworfenen OCT-Lichts orthogonal zu dem linearen Polarisationszustand des Lichts ist, das aus dem Austrittsende 45 des zweiten Lichtleiters 44 austritt. Dies ist typischerweise der Fall, wenn der Winkel zwischen linearer Polarisation am Eintritt in das Plättchen und der kristalloptischen Achse des Plättchens 45° beträgt., Aufgrund des orthogonalen Polarisationszustands tritt das zurückgeworfene OCT-Licht in die langsame Achse des zweiten polarisationserhaltenden Lichtleiters 44 ein.

Der Objektstrahlengang 23 des von dem Messobjekt 14 zurückgeworfenen OCT-Lichts setzt sich durch den ersten Faserkoppler 40 hindurch fort in einen vierten polarisationserhaltenden Lichtleiter 46, der sich zwischen dem ersten Faserkoppler 40 und einem zweiten Faserkoppler 47 erstreckt. Der vierte polarisationserhaltende Lichtleiter 46 ist so an den ersten Faserkoppler 40 angeschlossen, dass das OCT-Licht aus der langsamen Achse des zweiten Lichtleiters 44 in die langsame Achse des dritten Lichtleiters 46 übergeht.

Der Referenzstrahlengang 24 erstreckt sich durch einen dritten polarisationserhaltenden Lichtleiter 48, der zwischen dem ersten Faserkoppler 40 und dem zweiten Faserkoppler 47 angeordnet ist und der in einen ersten Abschnitt 49 und einen zweiten Abschnitt 50 unterteilt ist. Der erste Abschnitt 49 und der zweite Abschnitt 50 sind in einem Faserverbinder 51 miteinander verbunden, wobei der zweite Abschnitt 50 um 90° relativ zu dem ersten Abschnitt 49 verdreht ist.

Das von der OCT-Lichtquelle 16 kommende Licht wird in dem ersten Faserkoppler 40 in die Achse des ersten Abschnitts 49 des dritten polarisationserhaltenden Lichtleiters 48 eingeleitet. In dem Faserverbinder 51 erfolgt eine Übergabe in die langsame Achse des zweiten Abschnitts 50 des dritten Lichtleiters 48. Die Länge des ersten Abschnitts 49 entspricht der Länge des zweiten Lichtleiters 44, so dass das OCT-Licht im Objektarm und im Referenzarm dieselbe Wegstrecke in der schnellen Achse zurücklegt. Die Länge des zweiten Abschnitts 50 des dritten Lichtleiters 48 entspricht der Summe der Längen des zweiten Lichtleiters 44 und des vierten Lichtleiters 46, so dass die in der langsamen Achse zurückgelegte Wegstrecke im Referenzarm und im Objektarm gleich ist. Durch die Länge des ersten Abschnitts 49 lässt sich die Lage des Referenzpunkts im Objektstrahlengang wählen. Die optische Länge des ersten Abschnitts 49 muss dann der Summe der optischen Länge des zweiten Lichtleiters 44 und der doppelten optischen Weglänge vom Austrittspunkt 45 bis zum Referenzpunkt entsprechen.
In dem zweiten Faserkoppler 47 entsteht das Interferenzsignal aus dem Objektstrahlengang 23 und dem Referenzstrahlengang 24. Mit zwei Detektoren 52, 53 werden die um 180° phasenverschobenen Interferenzsignale aus dem zweiten Faserkoppler 47 aufgenommen. Durch Differenzbildung zwischen den beiden Detektoren 52, 53 kann der stationäre Anteil des Signals eliminiert werden, so dass sich ein Nutzsignal mit hoher Auflösung ergibt. Die Differenz der Photoströme der Detektoren 52, 53 wird in eine Spannung gewandelt und digitalisiert. Dabei wird das Interferenzsignal für einen Durchstimmvorgang der OCT-Lichtquelle 16 zunächst spektral aufgelöst digitalisiert und anschließend in ein räumliches Signal transformiert. Durch laterale Ablenkung des OCT-Strahls mit der Scaneinrichtung 27, 28 können Schnittbilder des Messobjekts 14 erstellt werden.

Auch bei dieser Ausführungsform haben die von dem Messobjekt 14 zurückgeworfenen Anteile des OCT-Lichts und die parasitären Reflexionen an den optischen Elementen des Objektarms 19 zueinander orthogonale Polarisationszustände. Das in der langsamen Achse des dritten Lichtleiters 46 transportierte OCT-Licht trägt maximal zu dem Interferenzsignal in dem zweiten Faserkoppler 47 bei, während die in der schnellen Achse transportierten parasitären Reflexionen sich nur minimal in dem Interferenzsignal wiederfinden. Die Ausführungsform gemäß Fig. 2 hat den Vorteil, dass durch die Verwendung der polarisationserhaltenden Lichtleiter 43, 44, 46, 48 unabhängig von dem Biegezustand der Lichtleiter 43, 44, 46, 48 der Polarisationszustand des OCT-Lichts erhalten bleibt. Die Elemente des OCT-Systems können also unter Verformung der Lichtleiter 43, 44, 46, 48 relativ zueinander bewegt werden, ohne dass das Interferenzsignal an Qualität verliert.

## Patentansprüche

1. OCT-System mit einer OCT-Lichtquelle (16) zum Aussenden von OCT-Licht (15) in einen Objektstrahlengang (23) und einen Referenzstrahlengang (24) und mit einem Detektor (25, 52, 53) zum Aufnehmen eines aus dem Objektstrahlengang (23) und dem Referenzstrahlengang (24) erzeugten Interferenzsignals, wobei in dem Objektstrahlengang (23) ein polarisationsabhängiges Verzögerungselement (30) angeordnet ist.

2. OCT-System nach Anspruch 1, **dadurch gekennzeichnet, dass** Licht aus dem Referenzstrahlengang (24) an einem Interferenz-Strahlenteiler (23, 40, 47) mit Licht aus dem Objektstrahlengang (23) zur Interferenz gebracht wird, wobei das polarisationsabhängige Verzögerungselement (30) so eingerichtet ist, dass am Interferenz-Strahlteiler (23, 40, 47) der Polarisations-Überlapp zwischen dem vom Messobjekt (14) zurückgestreuten Licht und dem aus dem Referenzstrahlengang (24) eintreffenden Licht größer ist als der Polarisations-Überlapp zwischen dem von einem oder mehreren optischen Elementen im Objektstrahlengang (23) reflektierten Licht und dem aus dem Referenzstrahlengang (24) eintreffenden Licht.

3. OCT-System nach Anspruch 1 oder 2, **dadurch gekennzeichnet, dass** das OCT-Licht (15) vor dem Eintreten in das Verzögerungselement (30) polarisiert ist.

4. OCT-System nach einem der Ansprüche 1 bis 3, **dadurch gekennzeichnet, dass** das polarisationsabhängige Verzögerungselement (30) so angeordnet ist, dass es von dem OCT-Licht (15) im Objektstrahlengang zweimal durchquert wird.

5. OCT-System nach einem der Ansprüche 1 bis 4, **dadurch gekennzeichnet, dass** das Verzögerungselement (30) ein λ/4-Plättchen ist.

6. OCT-System nach einem der Ansprüche 1 bis 5, **dadurch gekennzeichnet, dass** alle optischen Elemente, die den Objektstrahlengang formen oder umlenken, zwischen der OCT-Lichtquelle (16) und dem Verzögerungselement (30) angeordnet sind.

7. OCT-System nach einem der Ansprüche 1 bis 6, **dadurch gekennzeichnet, dass** eine dem Objekt (14) zugewandte Grenzfläche des polarisationsabhängigen Verzögerungselements (30) gegenüber dem Objektstrahlengang (23) verkippt ist.

8. OCT-System nach einem der Ansprüche 1 bis 7, **dadurch gekennzeichnet, dass** das OCT-Licht in einem ersten Strahlenteiler (18) in den Objektstrahlengang (23) und den Referenzstrahlengang (24) aufgespalten wird.

9. OCT-System nach Anspruch 8, **dadurch gekennzeichnet, dass** das Interferenzsignal in dem ersten Strahlenteiler (23, 40) erzeugt wird.

10. OCT-System nach Anspruch 8, **dadurch gekennzeichnet, dass** das Interferenzsignal in einem zweiten Strahlenteiler (47) erzeugt wird.

11. OCT-System nach Anspruch 10, **dadurch gekennzeichnet, dass** der Referenzstrahlengang (24) sich zwischen dem ersten Strahlenteiler (40) und dem zweiten Strahlenteiler (47) erstreckt.

12. OCT-System nach einem der Ansprüche 1 bis 11, **dadurch gekennzeichnet, dass** eine oder mehrere Lichtleiter (43, 44, 46, 50) des OCT-Systems als polarisationserhaltende Lichtleiter ausgebildet sind.

13. OCT-System nach Anspruch 12, **dadurch gekennzeichnet, dass** ein Abschnitt des Objektstrahlengangs (23) sich in einem polarisationserhaltenden Lichtleiter (44) erstreckt, wobei das OCT-Licht (15) sich auf dem Hinweg in der ersten Achse des polarisationserhaltenden Lichtleiters (44) ausbreitet und auf dem Rückweg in der zweiten Achse des polarisationserhaltenden Lichtleiters (44) ausbreitet.

14. OCT-System nach Anspruch 12 oder 13, **dadurch gekennzeichnet, dass** der Referenzstrahlengang (24) sich in einem polarisationserhaltenden Lichtleiter (49, 50) erstreckt, wobei in einem ersten Abschnitt (49) des Referenzstrahlengangs (24) das OCT-Licht (15) sich in der ersten Achse des polarisationserhaltenden Lichtleiters (49, 50) erstrecket und wobei in einem zweiten Abschnitt (50) des Referenzstrahlengangs (24) das OCT-Licht (15) sich in der zweiten Achse des polarisationserhaltenden Lichtleiters (49, 50) erstreckt.

15. OCT-Verfahren, bei dem OCT-Licht (15) ausgesendet wird und in einen Objektstrahlengang (23) und einen Referenzstrahlengang (24) aufgespalten wird, wobei mit einem Detektor (25, 52, 53,) ein aus dem Objektstrahlengang (23) und dem Referenzstrahlengang (24) erzeugtes Interferenzsignal aufgenommen und wobei das OCT-Licht (15) durch ein in dem Objektstrahlengang (23) angeordnetes polarisationsabhängiges Verzögerungselement (30) geleitet wird.
